# EUROPEAN PATENT APPLICATION

(11) **EP 1 614 413 A2**
(43) Date of publication of application: **11.01.2006**
(21) Application number: 05253780.0
(22) Date of filing: 17.06.2005
(51) Int. Cl.: A61K 9/28

(54) **Solid dosage form for acid-labile active ingredient**

(30) Priority: 18.06.2004 US 871851
(71) Applicant: McNeil-PPC, Inc., Skillman, NJ 08558 (US)
(72) Inventor: Shun-Pro, LI, Lansdale, PA 19446 (US); Wynn, David, Huntingdon Valley, PA 19006 (US); Sowden, Harry S, PA 19038 (US)
(74) Representative: Tunstall, Christopher Stephen

(57) **Abstract**

The present invention relates to solid, orally administrable dosage forms for acid-labile actives having at least one molded insert or core containing an acid-labile active ingredient, such as a proton pump inhibitor that is surrounded by barrier layer that is subsequently coated with an enteric layer. The present invention also relates to a dosage form that combines the barrier coated active ingredient containing insert with a second active ingredient.

## Description

### SUMMARY

The present invention relates to solid, orally administrable dosage forms having at least one molded insert or core containing an acid-labile active ingredient, preferably a proton pump inhibitor, that is surrounded by a barrier layer that is subsequently coated with a shell layer. Alternatively, the dosage form can combine the barrier coated active ingredient containing insert(s) with an antacid portion. The present invention discloses stable dosage forms for oral administration that comprises one or more of the acid-labile actives, especially benzimidazole derivatives omeprazole, lansoprazole or pantoprazole as an active ingredient as well as methods for their production.

### BACKGROUND

Acid-labile active ingredients, such as proton pump inhibitors, macrolide antibiotics, enzymes, and the like, must be specially formulated to pass through the stomach unharmed, then released in the intestinal tract where they may be absorbed into the general circulation. Typically pH-dependent coating materials have been used to accomplish this purpose, including polymeric materials that remain insoluble at gastric pH, then allow for release of active ingredient from a coated particle or coated dosage form at pH greater than about 5.0, e.g. 5.5, 6.0, 6.5, or 7.0. One problem with these particular materials, often referred to as "enteric coatings", is that they themselves tend to be acidic, and can contribute to the degradation of particularly acid-labile active ingredients.

One class of acid labile active ingredients is the group of pharmaceutical active ingredients called "proton pump inhibitors. These compounds, such as omeprazole, lansoprazole, rabeprazole, pantoprazole, and others listed below, are well-known treatments for gastric disorders. Chemical substances that degrade or are inactivated in an acid medium are termed herein acid-labile.

Many acid-labile compounds, such as the PPIs are most effective when made available in the weakly acidic to basic regions of the gastro-intestinal tract. In order to avoid contact between the substances and the acidic gastric fluid following oral administration of the substance, a pharmaceutical formulation is conventionally used, such as a capsule or tablet that contains a core having the acid-labile active substance and an outer layer that consists of a gastro-resistant, enter-soluble composition. Many enteric materials, however, present their own problems as they contain acidic groups that react with the acid-labile compounds. Significant research has been done to effectively combine enteric coatings and acid-labile actives to improve performance. Many attempts have been made to combine acid-labile actives for gastric disorders with other actives, such as antacids, analgesics and anti-inflammatories.

Dosage forms for acid-labile actives are known that provide for an active-containing core having an alkaline reacting compound, a protective subcoating and an enteric outer coating. Examples of such dosage forms are described in U.S. Patent No. 4,786,505 and U.S. Patent No. 4,853,230.

Many alternative dosage forms have been proposed in the literature.

U.S. Patent No. 5,232,706 describes formulations containing a nucleus formed by a mixture of a first basic compound and omeprazole, a first layer over the nucleus containing at least one basic water soluble excipient and a second basic compound, and an enteric coating.

U.S. Patent No. 5,626,875 describes formulations prepared by covering an inert nucleus with a first layer containing an acid-labile benzimidazole compound, a water soluble polymer and non-alkaline reacting excipients, an isolation layer and a final enteric coating.

U.S. Patent No. 5,753,265 and U.S. Patent No. 5,817,338 describe a multiple unit tablet having a multiple of core units (or pellets) having an active layer, wherein each core or pellet is covered with at least one enteric coating layer.

U.S. Patent No. 6,013,281 describes a process for preparing oral pharmaceutical formulations by forming a core material having a proton pump inhibitor and at least one alkaline reacting compound. A protective coating is formed in situ so as to surround the core material by reaction between the alkaline compound and the enteric coating polymer.

U.S. Patent No. 6,077,541, U.S. Patent No. 6,096,340 and U.S. Patent No. 6,174,548 describe pharmaceutical compositions of omeprazole having an inert core, a drug layer having an alkaline agent that surrounds the inert core, and an enteric coating layer that is applied directly onto the drug layer without a separating layer.

U.S. Patent No. 6,159,499 describes a composition substantially free of alkaline-reacting compounds comprising (a) a core containing an acid-labile benzimidazole active principle, wherein said core comprises a plurality of nuclei and said active principle mixed together and then compressed together, and wherein said active principle is not in the form of an alkaline salt, (b) an intermediate layer surrounding the core; and (c) an enteric layer surrounding the intermediate layer.

U.S. Patent No. 6,183,776 describes an oral pharmaceutical composition having a proton pump inhibitor and a separate second component selected from the group consisting of antacid agents, alginates and mixtures thereof. The composition is in the form of a multiple unit tablet, wherein the PPI is in the form of pellets covered with an enteric coating layer and the second component is separated from the first component by the enteric coating.

U.S. Patent No. 6,207,198 and U.S. Patent No. 6,248,355 describe capsules that contain "microtablets." Each microtablet has three component parts: (1) a core; (2) a subcoat; and (3) an enteric coat. The core contains an acid-labile active. The dosage form is exempt of an alkaline reacting compound.

U.S. Patent No. 6,224,910 describes a high drug load enteric coated pharmaceutical composition. The composition is in the form of beadlets having an enteric coating and a plasticizer but does not require a subcoat.

U.S. Patent No. 6,331,316 and U.S. Patent No. 6,569,457 describe an enteric-coated pharmaceutical composition comprising a core in the form of a tablet consisting essentially of an acid labile dosage form. An enteric coating surrounds the core and includes an alkalizing agent. No protective subcoat is provided between the enteric coating and the core.

U.S. Patent No. 6,428,810 describes an enteric coated pharmaceutical formulation having an active coated core, a very specific separating layer and an enteric coating layer.

U.S. Patent No. 6,489,346 describes a solid pharmaceutical composition in a dosage form that is not enteric-coated. The composition consists essentially of a selected non-enteric coated proton pump inhibitor and at least one buffering agent. A buffering agent is defined to be weak base or strong acid (and mixtures thereof) that, when formulated or delivered with the PPI, functions to substantially prevent or inhibit the acid degradation of the PPI by gastric acid sufficiently to preserve the bioavailability of the PPI administered. The buffering agent elevates the pH of the stomach sufficiently to achieve adequate bioavailability of the drug to effect therapeutic action.

U.S. Patent No. 6,602,522 describes a pharmaceutical composition having a tableted core having an uncoated granulation of active, an alkaline agent, at least one water soluble binder and at least one water insoluble binder, wherein a single coating comprising an enteric coating agent is provided around the tableted core.

US Patent No. 6,613,354 describes a capsule formulation that comprises a proton pump inhibitor, one or more non-steroidal anti-inflammatory drugs, an enteric coating layer, and optionally pharmaceutically acceptable excipients.

U.S. Patent No. 6,726,927 describes a process for manufacturing an oral pharmaceutical preparation of a core formulation comprising an acid-unstable drug and an alkaline substance, wherein the acid-unstable drug is omeprazole, sodium omeprazole, potassium omeprazole, lansoprazole, or a pharmaceutical salt of lansoprazole, the process consisting essentially of: (a) preparing the core formulation by dry mixing, without using an aqueous granulating solution, the acid-unstable drug with the alkaline substance; (b) quantitatively filling the core formulation into the hard gelatin capsule shell to give a filled hard gelatin capsule shell, wherein the gelatin capsule shell has an outer surface and an inner surface; and (c) coating the outer surface of the filled hard gelatin capsule shell with the enteric coating solution or dispersion.

WO 03/007917 is a published PCT application describing a multiparticulate tablet that disintegrates in the mouth. The tablet contains a proton pump inhibiting agent in the form of enteric coated microgranules and at least one antacid in the form of granules and a mixture of excipients comprising at least one disintegrating agent, one diluent and a lubricant. WO 03/063840 is a published PCT application describing a pharmaceutical composition having a core comprising an antacid and an outer layer that surrounds the core. The outer layer contains an effective amount of a proton pump inhibitor.

Published European Patent Application 247,983 discloses a pharmaceutical agent for oral administration that comprises omeprazole as an effective component. The core material contains omeprazole together with an alkaline reacting compound or an omeprazole salt, optionally together with an alkaline reacting adjuvant. Intermediate layers which form a separation layer between the alkaline reacting core and an outer layer of a gastric fluid-resistant coating comprise water-soluble tablet carrier mediums or tablet carrier mediums quickly disintegrating in water or polymeric, water-soluble, film-forming substance mixtures which optionally contain buffering, alkaline compounds.

Published European Patent Application 519,144 describes omeprazole pellets consisting of an inert pellet core that is coated with a micronized active ingredient and then subsequently coated with a gastric fluid-resistant layer.

Published European Patent Application 496,437 encompasses pellet cores and/or tablets that contain omeprazole or an alkaline salt of omeprazole together with an alkaline reacting compound and which are coated with a layer of water-soluble, film-forming adjuvants which preferably react with the alkaline as well as with a gastric fluid-resistant outer film.

Published European Patent Application 237,200 uses basic magnesium salts and/or basic calcium salts for stabilizing benzimidazole derivatives with omeprazole as a typical representative.

The dosage forms developed to date for delivering acid labile active ingredients typically require complex, time-intensive, and therefore costly manufacturing processes. There remains an un-met need for a dosage form design or construct that minimizes contact between an acid labile active ingredient and an acidic entero-soluble layer, yet can be manufactured using a less costly, less time intensive method.

### SUMMARY OF THE INVENTION

The present invention relates to a dosage form for oral administration for at least one solid insert comprising a matrix having an acid-labile pharmaceutical active ingredient distributed therein. A barrier layer is provided over the at least one insert that is substantially free of acid-labile pharmaceutical active ingredient and an enteric or gastric fluid-resistant layer that is provided over substantially the entire surface of the barrier layer. The terms "enteric" and "gastric fluid resistant" are, for purposes of this application, understood to equivalent. The barrier layer has a thickness of at least 200 microns at any point as measured from the outer surface of the at least one solid insert to the inner surface of the gastric fluid-resistant layer.

The barrier layer is preferably a compressed pharmaceutically acceptable powder material and/or substantially free of alkaline reacting compounds.

In one embodiment, at least one insert contains an acid-labile proton pump inhibitor, and preferably at least one insert is substantially free of alkaline reacting compounds.

The solid insert can contain a matrix having at least one acid-labile pharmaceutical active ingredient and a dissolution-enhancing vehicle. Alternatively, at least one insert consists essentially of a solid dispersion containing an acid-labile pharmaceutical active ingredient.

The present invention also relates to a dosage form for oral administration having at least one solid insert comprising a matrix having an acid-labile pharmaceutical active ingredient distributed therein and a barrier layer that is provided over the insert that is substantially free of acid-labile pharmaceutical active ingredient. An enteric or gastric fluid-resistant layer that is provided over substantially the entire surface of the barrier layer. The barrier layer has a minimum thickness that is greater than or equal to the smallest dimension of the at least one solid insert.

The present invention also relates to a dosage form for oral administration having a multi-compartment tablet comprising at least one solid insert comprising a matrix having an acid-labile pharmaceutical active ingredient, a barrier layer that surrounds the insert and is substantially free of acid-labile pharmaceutical active ingredient, an insulation layer that is provided over at least one surface of the barrier layer and a second active layer that is provided over the insulation layer. A coating is provided over the multi-compartment tablet that has at least one gastric fluid-resistant section and at least one non-gastric juice fluid-resistant section.

The present invention also relates to a method for production of a dosage form for oral administration comprising: (a) forming a molded article as a insert that contains an acid-labile active ingredient; (b) compressing a barrier layer around at least one insert containing the acid-labile active ingredient; and (c) applying a gastric fluid-resistant layer over the barrier layer. The barrier layer has a thickness of at least 200 microns at any point as measured from the outer surface of the at least one solid insert to the inner surface of the gastric fluid-resistant layer.

The present invention also relates to a method for production of a dosage form for oral administration comprising: (a) forming a multi-compartment core by (i) providing at least one molded article as a insert that contains an acid-labile active ingredient; (ii) compressing a barrier layer around at least one insert; (iii) applying an insulation layer over at least one surface of the barrier layer; (iv) providing a second active layer over the insulation layer; and (b) applying a coating over the multi-compartment core. The coating that is provided over the multi-compartment core has at least one gastric fluid-resistant section and at least one non-gastric juice fluid-resistant section.

The present invention relates to a dosage form, which upon oral ingestion provides delivery of a first active ingredient as substantially pH independent immediate release and delivery of an acid-labile pharmaceutical active ingredient by pH dependent delayed release.

The present invention further relates to a dosage form, which upon oral ingestion provides delivery an antacid by substantially pH independent immediate release and delivery of an acid-labile pharmaceutical active ingredient by pH dependent delayed release.

The dosage form of the present invention, upon contact with an appropriate dissolution medium, e.g. gastro-intestinal fluids, provides immediate release of a first active ingredient followed by a time delay, followed by release of an acid labile pharmaceutical active ingredient, only when the medium has reached a pH of at least about 5.0.

### BRIEF DESCRIPTION OF THE DRAWINGS

FIG. 1 shows a schematic layer construction according to the invention.
FIG. 2 shows a schematic layer construction of an alternative embodiment.

### DETAILED DESCRIPTION OF THE INVENTION

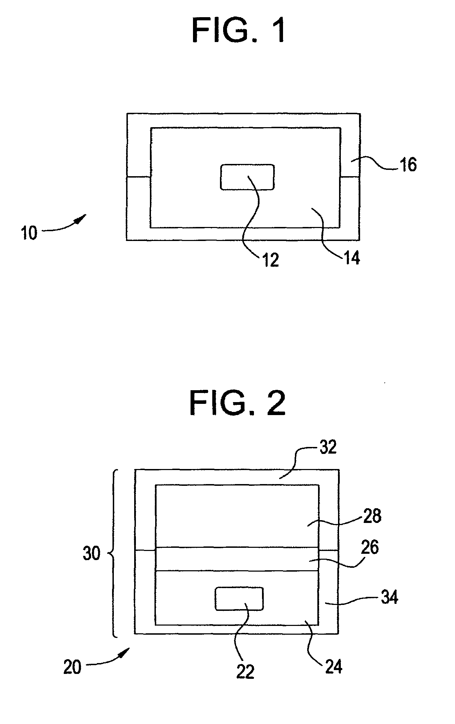

The layer construction of a dosage form 10 according to the invention is schematically given in FIG. 1. Dosage form 10 has an insert 12 that is embedded and surrounded by a barrier layer 14 that does not contain an alkaline reacting compound or buffering agent, as described herein. A shell 16 is provided over substantially the entire surface of barrier layer 14. Insert 12 of dosage form 10 contains an acid-labile active ingredient, such as proton pump inhibitors, including omeprazole, lansoprazole or pantoprazole, rabeprazole (Aciphex), pantoprazole (Protonix), esomeprazole magnesium (Nexium), individually or combinations thereof, proteins, didanosine, erythromycin, pancrelipase, amylase, cellulase, protease and lipase.

As used herein, the term "dosage form" applies to any solid object or semi-solid composition designed to contain a specific pre-determined amount (dose) of a certain ingredient, for example an active ingredient as defined below. Suitable dosage forms may be pharmaceutical drug delivery systems, including those for oral administration; or compositions for delivering minerals, vitamins and other nutraceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human. In another preferred embodiment, the dosage form is an orally administered "placebo" system containing pharmaceutically inactive ingredients, and the dosage form is designed to have the same appearance as a particular pharmaceutically active dosage form, such as may be used for control purposes in clinical studies to test, for example, the safety and efficacy of a particular pharmaceutically active ingredient.

The dosage form of the present invention preferably contains one or more active ingredients, in addition to the acid-labile active. Suitable active ingredients broadly include, for example, pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, oral contraceptives, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

One or more active ingredients of the present invention are considered to be "acid labile", that is they are either degraded or inactivated in an acid medium, such as gastric fluid. Acid labile pharmaceutical active ingredients useful in the present invention include those belonging to the pharmacological class of proton pump inhibitors, such as omeprazole, lansoprozole, rabeprazole, pantoprazole, esomeprazole, and the like and pharmaceutically acceptable salts esters and isomers thereof; some antibiotics, particularly the macrolide class, which includes erythromycin, clarithromycin, azithromycin, and the like and pharmaceutically acceptable salts, esters, isomers thereof; some antivirals, including the synthetic purine nucleoside analogue didanosine, which is active against the Human Immunodeficiency Virus (HIV); and enzymes such as pancrelipase, amylase, cellulase, protease, lipase, lactase, and the like. Those skilled in the art will recognize the above list of acid labile active ingredients is not exhaustive.

The active ingredient or ingredients are present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dosing regimen, the age and weight of the patient, and other factors must be considered, as known in the art. Typically, the dosage form comprises at least about 0.1 weight percent, preferably, the dosage form comprises at least about 1 weight percent, e.g. about 5 weight percent of a combination of one or more active ingredients.

In embodiments in which it is desired for at least one active ingredient to be absorbed into the systemic circulation of an animal, the active ingredient or ingredients are preferably capable of dissolution upon contact with a dissolution medium such as water, gastric fluid, intestinal fluid or the like.

Insert 12 of dosage form 10 is formed as a molded article or insert, preferably by injection molding with a flowable material. Preferred molded articles are pellet and microtablets.

Flowable materials, as the phrase is used herein, is meant to describe liquid materials, not powder compositions. It is understood that a flowable liquid material may contain undissolved solid particles wherein the flow characteristics of the overall composition is dictated primarily by the liquid carrier medium. Furthermore, a substance may be flowable only when heated to a particular temperature and/or when subjected to sufficient pressure. Consequently, the meaning of a flowable material must take into consideration the actual operating conditions of the equipments and processes.

In one embodiment, insert 12 of the present invention is made by molding, preferably using a solvent-free process. In this embodiment, the flowable material for making the insert comprises 10-100% by weight of a thermoplastic material or low melting hydrophobic material having a melting point of less than about 100°C, preferably from about 20 to about 100°C; and optionally up to about 30 weight percent of various adjuvants such as for example plasticizers, gelling agents, colorants, stabilizers, preservatives, and the like as known in the art. Insert 12 can optionally further comprise up to about 55 weight percent of one or more release-modifying excipients as described below. Insert 12 is preferably substantially free of alkaline reacting compounds or buffering agents, each defined herein. Substantially free means that the material can contain alkaline impurities or minor amounts of alkaline reacting compounds that do not materially affect their properties.

In one preferred embodiment, insert 12 comprises a thermoplastic material selected from polycaprolactones, polyvinyl acetate, polyalkylene glycols and combinations thereof at a level of about 30 to about 70 weight percent, e.g. about 35 to about 50 weight percent of the matrix. In one such embodiment, insert 12 further comprises a thermoplastic polyalkylene oxide at a level of about 15 to about 25% by weight as a strengthening polymer. Polyalkylene oxides having suitable thermoplastic properties for use in the present invention have a molecular weight of about 100,000 to about 900,000. In another such embodiment, insert 12 is substantially free of poly(alkylene oxide), e.g. contains less than 1%, or contains less than 0.1 weight percent of poly(ethylene oxide). The term matrix, as used in this application, is a mixture of inactive carrier materials throughout which the pharmaceutically active ingredients or active-containing components are distributed.

In one embodiment of the invention, insert 12 is made by the thermal setting molding method and apparatus described in U.S. Patent Application 2003-0124183 A1, published July 3, 2003 or alternatively in copending U.S. patent application Serial No. 10/677,984, filed October 2, 2003, the disclosures of which are each incorporated herein by reference. In this embodiment, insert 12 is formed by injecting a starting material in flowable form into a molding chamber. In one embodiment, the starting material comprises at least one active ingredient and a thermoplastic material at a temperature above the melting point of the thermoplastic material but below the decomposition temperature of the acid-labile active ingredient. In another embodiment, the active ingredient may be added to the starting material in the molding chamber via a separate injection. The starting material is cooled and solidifies in the molding chamber into a shaped form (i.e., having the shape of the mold).

The flowable material can be any edible material that is flowable at a temperature between about 37°C and 250°C, and that is solid, semi-solid, or can form a gel at a temperature between about -10°C and about 35°C. When it is in the fluid or flowable state, the flowable material can comprise a dissolved or molten component, and optionally a solvent, such as, for example, water or organic solvents, or combinations thereof. The solvent can be partially or substantially removed by drying. The flowable material can have, in addition to the at least one acid-labile active, which is desirable to incorporate into a shaped form, other functional ingredients, such as nutritionals, vitamins, minerals, flavors, sweeteners, and the like. Preferably, the starting material comprises at least one acid-labile active in micronized form and a thermoplastic material or low melting hydrophobic material.

Suitable flowable materials in a dissolved state for making the insert thereof by molding include those comprising thermoplastic materials, low melting hydrophobic materials such as fats and waxes, and the like. Suitable molten forms of flowable material include thermoplastic materials, low melting hydrophobic materials and the like. Suitable dissolved components for the flowable material include film formers, thickeners, such as gelling polymers or hydrocolloids, non-crystallizable carbohydrates, and the like.

Suitable thermoplastic materials that can be molded and shaped when heated include, water-soluble, water swellable and water insoluble polymers that are generally linear, or not crosslinked or strongly hydrogen bonded to adjacent polymer chains. Examples of suitable thermoplastic materials include: thermoplastic water swellable cellulose derivatives, thermoplastic water insoluble cellulose derivatives, thermoplastic vinyl polymers, thermoplastic starches, thermoplastic polyalkylene glycols, thermoplastic polyalkylene oxides, and amorphous sugar-glass, and the like, and derivatives, copolymers, and combinations thereof. As used herein, the term water swellable, as applied to polymers, includes polymers that form dispersions at the molecular level in water (sometimes referred to as "water-soluble" polymers), as well as those that hydrate and swell in an aqueous environment.

Examples of suitable thermoplastic water swellable cellulose derivatives include hydroxypropyl cellulose (HPC), hydroxypropylmethylcellulose (HPMC), methyl cellulose (MC). Examples of suitable thermoplastic water insoluble cellulose derivatives include cellulose acetate (CA), ethyl cellulose (EC), cellulose acetate butyrate (CAB), and cellulose propionate.

Examples of suitable thermoplastic vinyl polymers include polyvinyl alcohol (PVA) and polyvinyl pyrrolidone (PVP).

Examples of suitable thermoplastic starches are disclosed for example in U.S. Patent No. 5,427,614.

Examples of suitable thermoplastic polyalkylene glycols include polyethylene glycol. Examples of suitable thermoplastic polyalkylene oxides include polyethylene oxide having a molecular weight from about 100,000 to about 900,000 Daltons.

Other suitable thermoplastic materials include sugar in the form on an amorphous glass such as that used to make hard candy forms.

Any film former known in the art is suitable for use in the flowable material of the present invention. Examples of suitable film formers include, but are not limited to, film-forming water-soluble polymers, film-forming proteins, and film-forming water insoluble polymers.

Any thickener known in the art is suitable for use in the flowable material of the present invention. Examples of such thickeners include but are not limited to hydrocolloids (also referred to herein as gelling polymers), clays, gelling starches, and crystallizable carbohydrates, and derivatives, copolymers and mixtures thereof.

In one embodiment of the invention, the flowable material comprises gelatin as a gelling polymer. Gelatin is a natural, thermogelling polymer. It is a tasteless and colorless mixture of derived proteins of the albuminous class that is ordinarily soluble in warm water. Two types of gelatin - Type A and Type B - are commonly used. Type A gelatin is a derivative of acid-treated raw materials. Type B gelatin is a derivative of alkali-treated raw materials. The moisture content of gelatin, as well as its Bloom strength, composition and original gelatin processing conditions, determine its transition temperature between liquid and solid. Bloom is a standard measure of the strength of a gelatin gel, and is roughly correlated with molecular weight. Bloom is defined as the weight in grams required to move a half-inch diameter plastic plunger 4 mm into a 6.67% gelatin gel that has been held at 10°C for 17 hours. In a preferred embodiment, the flowable material is an aqueous solution comprising 20% 275 Bloom pork skin gelatin, 20% 250 Bloom bone gelatin, and approximately 60% water.

In a particularly preferred embodiment, the flowable material contains a thermoplastic material comprising water-soluble polyethylene glycol (PEG 3350, which is commercially available) or a glycerol ester of fatty acids (Gelucire 44/14, which is a saturated polyglycolized glyceride that is commercially available) and mixtures thereof (most preferably) that forms a solid dispersion. One such composition contains polyethylene glycol at a level of 5 to about 80 weight percent, e.g. about 20 to about 65 weight percent of the matrix, and glycerol ester of fatty acids at a level of 5 to about 60 weights percent, e.g. about 20 to about 40 weight percent of the matrix.

The active ingredient is intimately mixed with a dissolution-enhancing dispersion vehicle e.g. polyalkylene glycols (PEG and the like), emulsifiers and other amphiphilic materials, low melting hydrophilic materials, etc. One particularly useful dispersion vehicle is generically called "Lauroyl Macrogolglycerides", and available from Gattefosse S.A., France under the tradename "Gelucire". Lauroyl Macrogolglycerides are a combination of polyethylene glycol, fatty acid esters of polyethylene glycol, and mono-, di-, and tri- glycerides. In certain embodiments, the active ingredient may optionally be held in an amorphous state in the dispersion. In such embodiments, the amorphous active ingredient has a faster dissolution rate than its more thermodynamically stable crystalline form. In certain other embodiments, the dispersion vehicle serves to maintain a uniform dispersion of small particles and prevent crystal growth. In such embodiments, the relatively high surface area of the small crystals or particles serves to maximize the dissolution rate of the active ingredient. In particular embodiments in which the active ingredient and dispersion vehicle form a "solid solution", the active ingredient and dispersion vehicle are intimately mixed, e.g. dispersed at the molecular level, as evidenced by Differential Scanning Calorimetry methods well known in the art. Solid dispersions, including solid solutions, have conventionally been employed for the purpose of increasing dissolution rate of poorly soluble active ingredients.

Insert 12 is surrounded by barrier layer 14. Barrier layer 14 is preferably composed of materials that form a visually distinct or readily identifiable layer relative to insert 12. Barrier layer 14 may be formed by any method, including compression, molding, dipping, or spray coating. In one particular embodiment, in which the insert 12 is prepared by molding, and barrier layer 14 is prepared by compression, insert 12 is, subsequent to the molding process, introduced into a powder bed on a tablet compression machine, and compression-coated with a barrier layer 14. Insert 12 can be transferred from a molding module to a compression module using a transfer mechanism as described in published U.S. Patent application, published as US 2003-0068367A1 on April 10, 2003, which is incorporated herein by reference. The barrier layer can be applied by compression using the compression module in U.S. Patent Application 2003-0124183 A1, published July 3, 2003, the disclosure of which is incorporated herein by reference.

Barrier layer 14 has a layer thickness of approximately 200 to 30,000 microns, e.g. 500 to 3000 microns, say at least about 500 microns, or at least about 1000 microns on at least a portion (preferably a majority of its surface area as measured perpendicularly from the surface) of insert 12 and forms a mechanical as well as chemical barrier for insert 12. The thickness can vary depending on the size of the dosage form, the shape of insert 12, shape of barrier layer 14, and the relative positioning of the two components. In certain embodiments, barrier layer 14 has a thickness that is at least about 100%, e.g. at least about 150%, say at least about 200% of the smallest dimension (length, width, diameter) of the insert. In a preferred embodiment, barrier layer 14 is compressed and can have any shape that can be compressed. Thickness is measured as the shortest distance from one surface to surface of an adjacent layer. If the dosage form contains a plurality of inserts, the distance is measured from the surface of the insert closest to the adjacent layer.

Suitable shapes for compressed dosage forms include tablet shapes formed from compression tooling shapes described by "The Elizabeth Companies Tablet Design Training Manual" (Elizabeth Carbide Die Co., Inc., p.7 (McKeesport, Pa.) (incorporated herein by reference) as follows (the tablet shape corresponds inversely to the shape of the compression tooling):

| | | | |
|---|---|---|---|
| Shallow Concave. | Standard Concave. | Deep Concave. | Extra Deep Concave. |
| Modified Ball Concave. | Standard Concave Bisect. | Standard Concave Double Bisect. | Standard Concave European Bisect. |
| Standard Concave Partial Bisect. | Double Radius. | Bevel & Concave. | Flat Plain. |
| Flat-Faced-Beveled Edge (F.F.B.E.). | F.F.B.E. Bisect. | F.F.B.E. Double Bisect. | Ring. |
| Dimple. | Ellipse. | Oval | Capsule. |
| Rectangle. | Square. | Triangle. | Hexagon. |
| Pentagon. | Octagon. | Diamond. | Arrowhead. |
| Bullet. | Barrel. | Half Moon. | Shield. |
| Heart. | Almond. | House/Home Plate. | Parallelogram. |
| Trapezoid. | Figure 8/Bar Bell. | Bow Tie. | Uneven Triangle. |

The surface of one or more faces of barrier layer 14 can be substantially smooth, i.e. can have indentations or protrusions only at the microscopic level on the order of less than about 20 microns in width, depth, or height. Alternately the surface of one or more faces barrier layer 14 can be textured, i.e. having indentations or protrusions greater than about 20 microns, e.g. greater than about 50 microns, or greater than about 100 microns, or from about 1000 microns to about 30,000 microns in width, depth, or height.

In embodiments wherein the surface of one or more faces of barrier layer 14 is textured, the surface can contain an embossed (raised) or debossed (indented) design. For example, the surface of one or more faces barrier layer 14 can contain indentations, intagliations, letters, symbols or a pattern such as a graphic or logo. Alternatively, one or more faces of barrier layer can contain one or more depressions covering a substantial proportion of its surface area, for example at least about 10%, or at least about 20% or at least about 30% or at least about 50% of the surface area of the face. One type of compressed tablets with indentations in a major face is described for example in WO 01/85437, which describes a process for the production of tablets with a cavity using a press and is incorporated herein by reference.

Suitable excipients for barrier layer 14 include fillers, binders, disintegrants, lubricants, glidants, and the like, as known in the art.

Suitable fillers for use in making the barrier layer, or a portion thereof, by compression include water-soluble compressible carbohydrates such as sugars, which include dextrose, sucrose, maltose, and lactose, sugar-alcohols, which include mannitol, sorbitol, maltitol, xylitol, starch hydrolysates, which include dextrins, and maltodextrins, and the like, water insoluble plastically deforming materials such as microcrystalline cellulose or other cellulosic derivatives, water-insoluble brittle fracture materials such as dicalcium phosphate, tricalcium phosphate and the like and mixtures thereof.

In certain preferred embodiments, the filler for making the barrier layer is selected from water-soluble compressible carbohydrates, and water insoluble plastically deforming materials. In certain embodiments, the barrier layer 14 may comprise filler at a level of at least about 90%, e.g. at least about 95%, say at least about 98% by weight of the barrier layer. Lactose is particularly preferred.

Suitable binders for making the barrier layer, or a portion thereof, by compression include dry binders such as PVP, HPMC, and the like; wet binders such as water-soluble polymers, including hydrocolloids such as acacia, alginates, agar, guar gum, locust bean, carrageenan, carboxymethylcellulose, tara, gum arabic, tragacanth, pectin, xanthan, gellan, gelatin, maltodextrin, galactomannan, pusstulan, laminarin, scleroglucan, inulin, whelan, rhamsan, zooglan, methylan, chitin, cyclodextrin, chitosan, PVP, cellulosics, sucrose, starches, and the like; and derivatives and mixtures thereof.

Suitable disintegrants for making the barrier layer or a portion thereof, by compression, include sodium starch glycolate, cross-linked polyvinylpyrrolidone, cross-linked carboxymethylcellulose, starches, microcrystalline cellulose, and the like.

Suitable lubricants for making the barrier layer, or a portion thereof, by compression include long chain fatty acids and their salts, such as magnesium stearate and stearic acid, talc, glycerides and waxes.

Suitable glidants for making the barrier layer, or a portion thereof, by compression, include colloidal silicon dioxide, and the like.

Suitable release-modifying compressible excipients for making the barrier layer, or a portion thereof, by compression include swellable erodible hydrophilic materials, insoluble edible materials, pH-dependent polymers, and the like.

The intermediate product comprising insert 12 and barrier layer 14 is surrounded by a shell coating 16 for the production of one embodiment of the dosage form according to the invention. The shell may be applied by any suitable method, including molding, dipping, enrobing, compression coating, or spray coating. In certain embodiments the shell may be formed over the barrier layer, while in others the shell may be formed first, and the barrier layer added to the shell. Optionally, one or more intermediate film, i.e. "subcoating" layers (not shown) can be applied over barrier layer 14 and under shell coating 16.

Shell coating 16 represents a customary enteric, gastric fluid-resistant layer. Suitable film-forming polymers for the enteric, gastric fluid resistant layer include enteric cellulose derivatives, such as for example hydroxypropyl methylcellulose phthalate, hydroxypropyl methylcellulose acetate succinate, cellulose acetate phthalate; natural resins, such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename, EUDRAGIT S, and poly(methacrylic acid, methyl methacrylate) 1:1, which is commercially available from Rohm Pharma GmbH under the tradename, EUDRAGIT L, and the like, and derivatives, salts, copolymers, and combinations thereof. In certain embodiments, one preferred enteric composition is shellac and can be applied conventionally by pan coating or spray drying, or injection molding.

The compositions and resulting dosage forms described herein can preferably eliminate any alkaline reacting compounds or buffering agents in any layers or components of the dosage form which would create an alkaline microenvironment around the acid-labile active during use. An alkaline reacting compound is, for purposes of this application, a compound that creates a micro-environment around the acid-labile active when in contact with an acid or neutral aqueous medium to create a microenvironment having a pH above 7. Such substances can be chosen among, but are not restricted to substances such as the sodium, potassium, calcium, magnesium and aluminum salts of phosphoric acid, carbonic acid, citric acid or other suitable weak inorganic or organic acids.

Buffering agents are known in the art and can overlap with the compounds expected to function as alkaline reacting compounds. Examples of buffering agents include, but are not limited to, sodium bicarbonate, potassium bicarbonate, magnesium hydroxide, magnesium lactate, magnesium gluconate, magnesium oxide, magnesium aluminate, magnesium carbonate, magnesium silicate, magnesium citrate, aluminum hydroxide, aluminum phosphate, aluminum hydroxide/magnesium carbonate, potassium carbonate, potassium citrate, aluminum hydroxide/sodium bicarbonate, calcium chloride and calcium hydroxide. Sufficient quantities could allow a buffering agent to function as an alkaline reacting compound.

An alternative embodiment of the present invention is multi-compartment dosage form 20 shown in Figure 2 having 2 or more active ingredients that are preferably kept separated in discrete compartments of the dosage form. Preferably the dosage form contains a first, acid-labile ingredient, and a second active ingredient. The combination of an acid-labile active ingredient, such as the benzimidazole derivatives, and an antacid agent is particularly preferred.

Suitable active ingredients for use as the second active ingredient in the dosage form of the present invention broadly include, for example, pharmaceuticals, minerals, vitamins and other nutraceuticals, oral care agents, flavorants and mixtures thereof. Suitable pharmaceuticals include those listed above.

Suitable flavorants include menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

In one embodiment of the invention, the second active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the second active ingredient is selected from analgesics, anti-inflammatories, and antipyretics, e.g. non-steroidal anti-inflammatory drugs (NSAIDs), including a) propionic acid derivatives, e.g. ibuprofen, naproxen, ketoprofen and the like; b) acetic acid derivatives, e.g. indomethacin, diclofenac, sulindac, tolmetin, and the like; c) fenamic acid derivatives, e.g. mefenamic acid, meclofenamic acid, flufenamic acid, and the like; d) biphenylcarbodylic acid derivatives, e.g. diflunisal, flufenisal, and the like; e) oxicams, e.g. piroxicam, sudoxicam, isoxicam, meloxicam, and the like; f) cyclooxygenase-2 (COX-2) selective NSAIDs; and g) pharmaceutically acceptable salts of the foregoing.

Examples of useful propionic acid derivatives include ibuprofen, naproxen, benoxaprofen, naproxen sodium, fenbufen, flurbiprofen, fenoprofen, fenbuprofen, ketoprofen, indoprofen, pirprofen, carpofen, oxaprofen, pranoprofen, microprofen, tioxaprofen, suprofen, alminoprofen, tiaprofenic acid, fluprofen, bucloxic acid, and pharmaceutically acceptable salts, derivatives, and combinations thereof. In one embodiment of the invention, the propionic acid derivative is selected from ibuprofen, ketoprofen, flubiprofen, and pharmaceutically acceptable salts and combinations thereof. In another embodiment, the propionic acid derivative is ibuprofen, 2-(4-isobutylphenyl) propionic acid, or a pharmaceutically acceptable salt thereof, such as the arginine, lysine, or histidine salt of ibuprofen. Other pharmaceutically acceptable salts of ibuprofen are described in US Patent Nos. 4,279,926, 4,873,231, 5,424,075 and 5,510,385, the contents of which are incorporated by reference.

In another particular embodiment of the invention, the second active ingredient may be an analgesic selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another particular embodiment of the invention, the second active ingredient may be selected from pseudoephedrine, phenylpropanolamine, chlorpheniramine, dextromethorphan, diphenhydramine, astemizole, terfenadine, fexofenadine, loratadine, desloratadine, cetirizine, mixtures thereof and pharmaceutically acceptable salts, esters, isomers, and mixtures thereof.

In another particular embodiment, the second active ingredient is an NSAID and/or acetaminophen, and pharmaceutically acceptable salts thereof.

Dosage form 20 contains at least one insert 22 having substantially the same features as insert 12 described above. Insert 22 is embedded in and/or surrounded by a barrier layer 24 having substantially the features as barrier layer 14 described above. Barrier layer 24 is preferably composed of materials that form a visually distinct or readily identifiable layer relative to insert 22. Insulation layer 26 represents a mechanical barrier, and remains intact for greater than one hour, e.g. greater than 2 hours, say about greater than 4 hours after contact with dissolution media. It could be erodible in an aqueous environment, or could be insoluble in an aqueous environment, but should not be readily soluble in an aqueous environment. Preferably the solubility of the insulation layer is pH independent.

An intermediate product comprising insert 22 and barrier layer 24 is made by any suitable method, in substantially the same manner as described above. The intermediate product can be provided, as noted above, with one or more optional subcoatings (not shown) over barrier layer 24. Subsequently, in one preferred embodiment, the intermediate product is provided via a tableting process with an insulation layer 26 covering at least one major surface, preferably exactly one major surface, of barrier layer 24 followed by an second active ingredient layer 28. In another preferred embodiment, the insulation layer 26 is applied to one surface of the barrier layer 24 via any suitable method such as molding or dipping. In this embodiment, the second active ingredient layer 28 may be applied by any suitable method, such as molding or compression. Second active ingredient layer 28 is provided over substantially all of insulation layer 26 but is not in communication with or otherwise contact barrier layer 24 prior to introducing the dosage form into a dissolution medium.

In one embodiment, insulation layer 26 contains water-soluble or water-swellable polymers that are erodible in aqueous media. Upon contact with an aqueous medium, water-soluble and/or water-swellable polymer in insulation layer 26 absorbs water and swells to form a gel layer to create a network or polymer matrix. As a function of time, the water soluble/swellable polymer gradually erodes from the top layer of the polymer matrix. Eventually, insulation layer 26 is sufficiently eroded to expose barrier layer 24, followed by release of insert 22. Preferably, the dissolution rate of insulation layer 26 is pH independent. Preferably, insulation layer 26 remains intact at least as long as enteric section 34.

In another embodiment, insulation layer 26 is composed primarily of water insoluble polymers to form a physical barrier that prevents barrier 24 and insert 22 from disintegrating in either acidic or alkaline aqueous environment.

In embodiments in which insulation layer 26 functions either by an erosion-based mechanism or as an insoluble barrier mechanism to provide a time delay for the release of an active ingredient from an insert 22, insulation layer 26 preferably comprises a release modifying excipient selected from swellable erodible hydrophilic materials, insoluble edible materials, insoluble material and combinations thereof.

Suitable swellable erodible hydrophilic materials for use in making the insulation layer include: water swellable cellulose derivatives, polyalkylene glycols, thermoplastic polyalkylene oxides, acrylic polymers, hydrocolloids, clays, gelling starches, and swelling cross-linked polymers, and derivatives, copolymers, and combinations thereof.

Examples of suitable water swellable cellulose derivatives include sodium carboxymethylcellulose, cross-linked HPC, HPC, HPMC, hydroxyisopropylcellulose, hydroxybutylcellulose, hydroxyphenylcellulose, HEC, hydroxypentylcellulose, hydroxypropylethylcellulose, hydroxypropylbutylcellulose, hydroxypropylethylcellulose.

Examples of suitable polyalkylene glycols include polyethylene glycol. Examples of suitable thermoplastic polyalkylene oxides include poly(ethylene oxide).

Examples of suitable acrylic polymers include potassium methacrylate divinylbenzene copolymer, polymethylmethacrylate, CARBOPOL (high-molecular weight cross-linked acrylic acid homopolymers and copolymers), and the like.

Examples of suitable hydrocolloids include alginates, agar, guar gum, locust bean gum, kappa carrageenan, iota carrageenan, tara, gum arabic, tragacanth, pectin, xanthan gum, gellan gum, maltodextrin, galactomannan, pusstulan, laminarin, scleroglucan, gum arabic, inulin, pectin, gelatin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, swelling starches such as sodium starch glycolate, and derivatives thereof. Examples of suitable swelling cross-linked polymers include cross-linked PVP, cross-linked agar, and cross-linked carboxymethylcellose sodium.

Suitable insoluble edible materials for use in making insulation layer 26 include water-insoluble polymers and low-melting hydrophobic materials. Examples of suitable water-insoluble polymers include ethylcellulose, PVA, polyvinyl acetate, polycaprolactones, CA and its derivatives, acrylates, methacrylates, acrylic acid copolymers; and the like and derivatives, copolymers, and combinations thereof. Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono-, di-, and tri-glycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

The preferred composition for the erodible embodiment for insulation layer 26 is a combination of HPMC, HEC and lactose, based upon the total dry weight of the insulation layer, from about 10 percent to about 20 percent of the HPMC and from about 20 percent to about 40 percent of the HEC and from about 40 percent to about 60 percent of the lactose that can be applied conventionally by compression coating, or more preferably by direct compression. The preferred composition for the insoluble embodiment for insulation layer 26 is a combination of ethylcellulose, HPMC and hydrogenated castor oil, based upon the total dry weight of the insulation layer, from about 40 percent to about 60 percent of the ethylcellulose and from about 20 percent to about 40 percent of the HPMC and from about 10 percent to about 40 percent of the hydrogenated castor oil that can be applied conventionally by compression coating, or more preferably by direct compression.

In certain preferred embodiments, a variety of antacid agent(s) and/or alginates can be used as second active ingredient layer 28 according to the present invention. Such antacid agents include, for example, aluminum hydroxide, calcium carbonate, magnesium hydroxide, magnesium carbonate and aluminum magnesium hydroxide carbonate taken alone or in combinations with each other. The alginates can be an alginate selected from alginic acid or sodium alginate or other pharmaceutically acceptable alginate salts, hydrates, esters etc.

The combined multi-layer core that comprises insert 22, barrier layer 24, insulation layer 26 and antacid layer 28, is transferred to a molding system as described above for final molded coating 30. Coating 30 comprises at least two sections; a non-enteric section 32 and an enteric section 34.

Film forming agents suitable for use in making non-enteric section 32 are known in the art and can be for example HPMC, povidone, HEC, other modified celluloses known in the art, gelatin, polyacrylates, polymethacrylates, polymethyl/ethylmethacrylates, polyethylene glycol, polyethylene oxide and combinations thereof. The materials can be formulated to give an immediate release by forming a coat that dissolves quickly.

Non-enteric section 32 is readily soluble in gastrointestinal fluids. Such section will preferably be breached or dissolved within 30 minutes in 900 ml water or 0.1 N HCl, or phosphate buffer solution at 37°C with stirring by a USP type 2 dissolution apparatus (Paddle method) at 50 or 100 rpm.

Non-enteric section 32 preferably comprises materials that exhibit rapid dissolution in gastro-intestinal fluids and can be one or several inert water-soluble layers or layers that rapidly disintegrate in an aqueous medium, containing non-acid inert pharmaceutical excipients. This layer comprises at least one polymer conventionally used in applications where a film is provided by coating such as: sugars, polyethylene glycol, polyethylene oxide, polyvinylpyrrolidone, poly (vinyl alcohol), HPC, hydroxy methylcellulose, HPMC, etc. Non-enteric section 32 can additionally contain any one of the conventional pharmaceutical excipients relating to insert 12/22, or a mixture thereof, and most notably silicon dioxide. Silicon dioxide is, when present, provided in an amount that can vary from between 2 and 45% by weight based on the dry weight of barrier layer 24, preferably 5 to 18% by weight, for example about 9%.

In certain other such embodiments, suitable film formers can be selected from film forming water soluble polymers such as for example water soluble vinyl polymers, water soluble polycarbohydrates, and water soluble copolymers; film-forming proteins, and combinations thereof.

In one embodiment, non-enteric section 32 preferably comprises at least about 50%, preferably at least about 80%, most preferably at least about 90% of a material selected from film formers, gelling polymers, low-melting hydrophobic materials, non-crystallizable sugars or sugar alcohols, and mixtures thereof. In another embodiment, non-enteric section 32 comprises at least about 50%, preferably at least about 80%, most preferably at least about 90% of a material selected from film formers, gelling polymers, low-melting hydrophobic materials, and mixtures thereof. In another particular embodiment, non-enteric section 32 comprises less than about 50%, preferably less than about 25%, most preferably less than about 5% of a crystallizable sugar.

In another embodiment, the dosage form is substantially free (i.e. less than 1% by weight, preferably less than about 0.1% by weight, based upon the shell weight) of charge control agents. As used herein, the term "charge control agents" refers to a material having a charge control function, such as those used for electrostatic deposition of coatings onto substrates. Such charge control agents include metal salicylates, for example zinc salicylate, magnesium salicylate and calcium salicylate; quaternary ammonium salts; benzalkonium chloride; benzethonium chloride; trimethyl tetradecyl ammonium bromide (cetrimide); and cyclodextrins and their adducts.

One preferred composition for non-enteric section 32 is a combination of polyethylene glycol and polyethylene oxide. Non-enteric section 32 typically comprises, based upon the total dry weight of the coating, from about 50 percent to about 90 percent of the polyethylene glycol and from about 1 percent to about 20 percent of the polyethylene oxide. Another preferred composition is a combination of gelatin and/or HPMC. The preferred composition typically comprises, based upon the total wet weight of the section, from about 15 percent to about 40 percent of the gelatin and from about 0 percent to about 20 percent of the HPMC and from about 20 percent to 80 percent of water and can be applied by injection molding.

Enteric section 34 has substantially the same features as the enteric coating 16 described above.

Non-enteric section 32 and enteric section 34 are preferably provided on dosage form 20 in a two stage injection molding process, such as the injection molding processes described hereinafter, such as the processes described in detail in copending application 10/677,984, filed October 2, 2003 or in U.S. Patent Application 2003-0124183 A1, published July 3, 2003, which are incorporated herein by reference.

The present invention further comprises a method for the production of a stable dosage form for oral administration, which comprises acid-labile actives as an active ingredient.

A solid, orally administrable dosage forms is produced according to the method of the invention for acid-labile actives having at least one insert containing an acid-labile active, such as a proton pump inhibitor, that is surrounded by barrier layer, which is subsequently coated with an enteric layer. The insert contains, for example, a proton pump inhibitor, such as omeprazole, and is made by molding, preferably using a solvent-free process. In a particularly preferred embodiment, the insert is composed of a flowable (under standard operating conditions) material comprising a blend of micronized omeprazole, polyethylene glycol and polyglycolyzed glycerides. Selected polyethylene glycol(s) and polyglycolyzed glyceride(s) are melted in a water-jacketed stainless steel vessel that is equipped with a mixer. The temperature of the vessel is maintained at 70°C throughout the process. Upon complete melting of the polymeric materials, micronized omeprazole powder is added to the molten mixture, while mixing continues until the micronized powder is uniformly dispersed.

The insert can be made by the thermal setting molding module in U.S. Patent Application 2003-0124183 A1, published July 3, 2003, the disclosure of which is incorporated herein by reference. Alternatively, the insert can be made using the injection module described in copending U.S. patent application Serial No. 10/677,984, filed October 2, 2003, the disclosure of which is incorporated herein by reference. The insert is, subsequent to the molding process, coated with a barrier layer. The barrier layer comprises a powder blend of a spray-dried mixture of crystalline and amorphous lactose monohydrate (commercially available as Fast-Flo from Foremost Farms) and magnesium stearate and is mixed using a stainless steel V blender to yield barrier layer blend for compression. The barrier layer is compressed using a round concave punch and die unit having 0.375" diameter. The barrier layer is applied by compression using the compression module in U.S. Patent Application 2003-0124183 A1, published July 3, 2003, the disclosure of which is incorporated herein by reference.

The core having an insert is made in such a way that the insert is approximately centered and is surrounded by the barrier layer. The intermediate product comprising the insert and the barrier layer is then coated with a shell coating. Shell coating represents a customary enteric, gastric fluid-resistant layer that comprises a flowable blend of polyethylene glycol, polyethylene oxide, triethyl citrate and shellac. Polyethylene glycol, polyethylene oxide, triethyl citrate and shellac are melted in a water-jacketed stainless steel vessel equipped with a mixer to make the flowable blend. The temperature of the vessel is maintained at 70°C throughout the manufacturing process. The shell coating is applied by injection molding using a thermal cycle molding module as described in U.S. Patent Application 2003-0124183 A1, published July 3, 2003, the disclosure of which is incorporated herein by reference or the injection molding module described U.S. patent application Serial No. 10/677,984, filed October 2, 2003.

Alternatively, the dosage form can combine the core having at least one insert containing active with an antacid portion. The dosage form is a stable medicament for oral administration that comprises one or more of the acid-labile actives, especially benzimidazole derivatives omeprazole, lansoprazole or pantoprazole as an active ingredient. In a preferred embodiment, the insert comprises a flowable blend of micronized omeprazole, polyethylene glycol and polyglycolyzed glycerides. The insert, subsequent to its molding process, is coated with a barrier layer. The barrier layer comprises a powder blend of a spray-dried mixture of crystalline and amorphous lactose monohydrate (commercially available as Fast Flo from Foremost Farms) and magnesium stearate and is mixed using a stainless steel V blender to yield barrier layer blend for compression. The barrier layer is compressed using a round concave punch and die unit having 0.375" diameter. The barrier layer is applied by compression using the compression module in U.S. Patent Application 2003-0124183 A1, published July 3, 2003, the disclosure of which is incorporated herein by reference.

The compression module is further modified to include two additional compression stations to permit production of a multi-layer core. The formula amount of the barrier layer blend is added to the die cavity in the first compression station, and then the insert is inserted into the blend. An insulation layer that comprises lactose, HPMC, HEC and magnesium stearate which are mixed using a stainless steel V blender to yield insulation layer blend for compression. The insulation layer blend is added onto core having an insert in the second compression station and gently tapped. A second active layer that comprises, for example, an antacid, such as calcium carbonate, croscramellose sodium and magnesium stearate are mixed using a stainless steel V blender to yield a second active layer blend for compression. The second active layer blend is added onto the insulation layer in the third compression station and compressed to yield the multi-layer core. The multi-layer core is then coated with two different shell portions.

The first shell portion represents a non-enteric layer that comprises a flowable blend of polyethylene glycol and polyethylene oxide. Polyethylene glycol and polyethylene oxide are melted to form the flowable blend in a water-jacketed stainless steel vessel equipped with a mixer. The temperature of the vessel is maintained at 70°C throughout the manufacturing process. The second shell portion represents a customary enteric, gastric fluid-resistant layer that comprises a flowable enteric blend of polyethylene glycol, polyethylene oxide, triethyl citrate and shellac. Polyethylene glycol, polyethylene oxide, triethyl citrate and shellac are melted in a water-jacketed stainless steel vessel equipped with a mixer to make the flowable enteric blend. The temperature of the vessel is maintained at 70°C throughout the entire manufacturing process.

Shell coatings described above are applied by injection molding using a thermal cycle molding module as described in U.S. Patent Application 2003-0124183 A1, published July 3, 2003, the disclosure of which is incorporated herein by reference or the injection molding module described in copending U.S. patent application Serial No. 10/677,984, filed October 2, 2003, the disclosure of which is incorporated herein by reference. The first shell portion represents a non-enteric layer is deposited onto the second active layer of the multi-layer core, whereas the second shell portion represents an enteric layer is deposited onto the core having an insert portion of the multi-layer core.

The novel dosage form described herein can most efficiently be produced using the injection molding processes described herein and has significant advantages over the known forms. Since the insert containing an acid-labile or a heat-labile active is inserted into the center of the barrier layer, the thick barrier layer prevents the active from contacting the enteric coating layer. Due to the separation, the dosage form does not require the incorporation of an alkaline reacting or buffering agent in the active-containing and/or barrier layer to prevent the active from experiencing chemical degradation.

The present invention is described in further detail by reference to the following non-limiting inventions. It will become apparent to those skilled in the art that various modifications to the preferred embodiments of the invention can be made by those skilled in the art without departing from the spirit or scope of the invention as defined by the appended claims.

### Example 1

Dosage forms according to the invention, comprising an insert containing a proton pump inhibitor that is surrounded by the barrier layer that is subsequently coated with an enteric layer, are prepared as follows.

The insert is made using the following ingredient:

| Ingredient | Trade Name | Manufacturer | Weight% | Mg/Dosage Form |
|---|---|---|---|---|
| Polyethylene Glycol 8000 | Carbowax® | Union Carbide Corp. Danbury, CT | 50.0 | 25 |
| Polyglycolyzed Glycerides | Gelucire® 44/14 | Gattefosse, Westwood, NJ | 30.0 | 15 |
| Micronized Omeprazole Powder | | Sigma, St. Louis, MO | 20.0 | 10 |

The insert is prepared as follows: a beaker is submersed in a 70°C water bath. Polyethylene glycol (PEG) 8000 and polyglycolyzed glycerides (Gelucire® 44/14) are added to the beaker and are mixed with a mixer until all of the PEG and polyglycolyzed glycerides are melted. Micronized omeprazole powder is added to the molten mixture and mixed until a uniform dispersion is achieved. The insert material is provided to the molding module in flowable form.

The insert is molded according to the following process: a laboratory scale (round, tablet-shaped, 0.1875" diameter) stainless steel mold assembly is used to make the insert. The mold assembly is comprised of a lower mold portion and an upper mold portion, each having no temperature control. Appropriate amounts of molten flowable material prepared above are introduced into mold cavity of both the lower and upper mold portion. The mold assembly is pressed together to form a mold insert. After 30 seconds, the upper mold portion is removed, and the molded insert is ejected from the lower mold portion.

The barrier layer is made using the following ingredient:

| Ingredient | Trade Name | Manufacturer | Weight% | Mg/Dosage Form |
|---|---|---|---|---|
| Lactose Monohydrate | Fast-flo lactose | Foremost Whey Products | 99.0 | 118.8 |
| Magnesium Stearate | | Mallinckrodt Inc., St. Louis, MO | 1.0 | 1.2 |

The barrier layer is made in the following manner: Fast Flo lactose and magnesium stearate are added to a plastic bag and shaken for 2 minutes to yield the barrier layer blend.

The barrier layer is applied onto the insert according to the following process: A model M hydraulic Carver Laboratory Press is employed. A round, concave punch and die unit having 0.375" diameter is used for the compression. A half formula amount of barrier layer blend is first filled into a die, and then the molded insert is manually placed in the center of the barrier layer blend. The remaining barrier layer blend is then poured into the die and is compressed at 3000 lb/square inch of operating pressure to prepare the core having an insert.

The gastric fluid-resistant shell layer is made using the following ingredient:

| Ingredient | Trade Name | Manufacturer | Weight % | Mg/Dosage Form |
|---|---|---|---|---|
| Polyethylene Glycol 8000 | Carbowax® | Union Carbide Corp. Danbury, CT | 45.0 | 64.8 |
| Shellac Powder | Regular bleached shellac | Mantrose-Haeuser Co., Atteboro, MA | 35.0 | 50.4 |
| Polyethylene Oxide (MW 300,000) | Polyox® WSR N-750 | Union Carbide Corp. Danbury, CT | 10.0 | 14.4 |
| Triethyl Citrate | | Morflex, Inc., Greensboro | 10.0 | 14.4 |

The gastric fluid-resistant shell layer dispersion is prepared as follows: a beaker is submersed in a 70°C water bath. Polyethylene glycol (PEG) 8000 and shellac powder are added to the beaker and are mixed with a mixer until all of the PEG and shellac are melted. The triethyl citrate is added to the molten mixture and mixed for two minutes. Polyethylene oxide (PEO) is added to the molten mixture and mixed for 15 minutes. The shell material is provided in flowable form.

The gastric fluid-resistant shell layer is applied onto the core having an insert according to the following process: A laboratory scale thermal cycle molding module as described in U.S. Patent Application 2003-0124183 A1, published July 3, 2003, the disclosure of which is incorporated herein by reference, having a diameter of 0.4219", is used to apply the first and second shell portions to the cores, and comprised of a single mold assembly made from an upper mold assembly portion comprising an upper mold cavity, and a lower mold assembly portion comprising a lower mold cavity.

The lower mold assembly portion is first cycled to a hot stage at 85°C for 30 seconds. The shell material is introduced into the lower mold cavity. A core having an insert prepared as described above is then inserted into the cavity. A blank upper mold assembly portion is mated with the lower mold assembly portion. The mold assembly is then cycled to a cold stage at 5°C for 60 seconds to harden the first shell portion. The blank mold assembly portion is removed from the lower mold assembly portion. The upper mold assembly portion is cycled to a hot stage at 85°C for 30 seconds. The shell material is added to the upper mold cavity. The lower mold assembly portion, which has been maintained at 5°C, is then mated with the upper mold assembly portion. The upper mold assembly portion is then cycled to a cold stage at 5°C for 120 seconds to harden the second shell portion. The lower mold assembly portion is then removed and the finished dosage form, a core having an insert coated with the molded gastric fluid-resistant shell layer, is ejected from the upper mold cavity. The weight gain due to the shell material (i.e. the difference in weight between the finished dosage form, and the core) is recorded.

### Example 2

Dosage forms according to the invention, comprising a tri-layer core having a first core portion containing a proton pump inhibitor that is surrounded by the barrier layer, a second core portion containing an insulation layer and a third core portion containing an antacid layer, within a shell comprising a first shell portion and a second shell portion are prepared as follows.

The insert for the first core portion is made using the following ingredient:

| Ingredient | Trade Name | Manufacturer | Weight% | Mg/Dosage Form |
|---|---|---|---|---|
| Polyethylene Glycol 8000 | Carbowax® | Union Carbide Corp. Danbury, CT | 50.0 | 25 |
| Polyglycolyzed Glycerides | Gelucire® 44/14 | Gattefosse, Westwood, NJ | 30.0 | 15 |
| Micronized Omeprazole Powder | | Sigma, St. Louis, MO | 20.0 | 10 |

The insert is prepared as follows: a beaker is submersed in a 70°C water bath. Polyethylene glycol (PEG) 8000 and polyglycolyzed glycerides (Gelucire® 44/14) are added to the beaker and mixed with a mixer until all of the PEG and polyglycolyzed glycerides are melted. Micronized omeprazole powder is added to the molten mixture and mixed until a uniform dispersion is achieved. The insert material is provided to the injection-molding module in flowable form.

The insert is molded according to the following process: a laboratory scale (round, tablet-shaped, 0.1875" diameter) stainless steel mold assembly is used to make the insert. The mold assembly is comprised of a lower mold portion and an upper mold portion, each having no temperature control. Appropriate amounts of flowable material are introduced into mold cavity of both the lower and upper mold portion. The mold assembly is pressed together to form a mold insert. After 30 seconds, the upper mold portion is removed, and the molded insert is ejected from the lower mold portion.

The barrier layer for the first core portion is made using the following ingredients:

| Ingredient | Trade Name | Manufacturer | Weight% | Mg/Dosage Form |
|---|---|---|---|---|
| Lactose Monohydrate | Fast Flo lactose | Foremost Whey Products | 99.0 | 118.8 |
| Magnesium Stearate | | Mallinckrodt Inc., St. Louis, MO | 1.0 | 1.2 |

The barrier layer is made in the following manner: Fast-flow lactose and magnesium stearate are added to a plastic bag and shaken for 2 minutes to yield the barrier layer blend.

The second core portion (insulation layer) is made using the following ingredients:

| Ingredient | Trade Name | Manufacturer | Weight% | Mg/Dosage Form |
|---|---|---|---|---|
| Hydroxypropyl Methylcellulose | Methocel K4M Perm CR | The Dow Chemical Co., Midland, MI | 15.0 | 9.0 |
| Hydroxyethyl cellulose | Klucel EXAF | Hercules Inc., Wilmington, DE | 30.0 | 18.0 |
| Lactose Monohydrate | Fast-flow lactose | Foremost Whey Products | 54.5 | 32.7 |
| Magnesium Stearate | | Mallinckrodt Inc., St. Louis, MO | 0.5 | 0.3 |

The insulation layer for the second core portion is made in the following manner: Lactose, hydroxypropyl methylcellulose and hydroxyethyl cellulose magnesium stearate are added to a plastic bag and shaken for 5 minutes, then magnesium stearate is added and shaken for 2 minutes to yield the erodible layer blend.

The third core portion (antacid layer) is made using the following ingredient:

| Ingredient | Trade Name | Manufacturer | Weight% | Mg/Dosage Form |
|---|---|---|---|---|
| Calcium Carbonate | DC Calcium Carbonate 90% AM | Watson Foods, Inc., West Haven, CT | 95.0 | 228.0 |
| Croscarmellose Sodium | Ac-Di-Sol® | FMC Corporation, Newark, DE | 4.5 | 10.8 |
| Magnesium Stearate | | Mallinckrodt Inc., St. Louis, MO | 0.5 | 1.2 |

The antacid layer is made in the following manner: Calcium carbonate and croscramellose sodium are added to a plastic bag and shaken for 5 minutes, then magnesium stearate is added to the plastic bag and shaken for 2 minutes to yield the antacid layer blend.

The tri-layer core is made according to the following process: A model M hydraulic Carver Laboratory Press is employed. A round, concave punch and die unit having 0.375" diameter is used for the compression. A half formula amount of barrier layer blend for the first core portion is first filled into a die, and then the molded insert containing omeprazole is manually placed in the center of the barrier layer blend.
The remaining barrier layer blend is then poured into the die and gently tapped. Then the formula amount of the insulation layer for the second core portion is fed into the cavity overlying the first core portion and gently tapped. Then the formula amount of the antacid layer for the third core portion is fed into the cavity overlying the second core portion and gently tapped. The granulation is compressed at 3000 lb/square inch of operating pressure to prepare the tri-layer core.

The gastric fluid-resistant shell layer for the first shell portion is made using the following ingredients:

| Ingredient | Trade Name | Manufacturer | Weight % | Mg/Dosage Form |
|---|---|---|---|---|
| Polyethylene Glycol 8000 | Carbowax® | Union Carbide Corp. Danbury, CT | 45.0 | 43.2 |
| Shellac Powder | Regular bleached shellac | Mantrose-Haeuser Co., Atteboro, MA | 35.0 | 33.6 |
| Polyethylene Oxide (MW 300,000) | Polyox® WSR N-750 | Union Carbide Corp. Danbury, CT | 10.0 | 9.6 |
| Triethyl Citrate | | Morflex, Inc., Greensboro | 10.0 | 9.6 |

The gastric fluid-resistant shell layer dispersion is prepared as follows: a beaker is submersed in a 70°C water bath. Polyethylene glycol (PEG) 8000 and shellac powder are added to the beaker and mixed with a mixer until all of the PEG and shellac are melted. The triethyl citrate is added to the molten mixture and mixed for two minutes. The polyethylene oxide (PEO) is added to the molten mixture and mixed for 15 minutes. The shell material is provided to the injection-molding module in flowable form.

The immediate release layer for the second shell portion is made using the following ingredient:

| Ingredient | Trade Name | Manufacturer | Weight % | Mg/Dosage Form |
|---|---|---|---|---|
| Polyethylene Glycol 8000 | Carbowax® | Union Carbide Corp. Danbury, CT | 85.0 | 81.6 |
| Polyethylene Oxide (MW 100,000) | Polyox® WSR N-10 | Union Carbide Corp. Danbury, CT | 15.0 | 14.4 |

The immediate release layer for the second shell portion is prepared as follows: a beaker is submersed in a 70°C water bath. Polyethylene glycol (PEG) 8000 is added to the beaker and mixed with a mixer until all of the PEG is melted. The polyethylene oxide (PEO) is added to the molten mixture and mixed for 15 minutes. The shell material is provided to the injection-molding module in flowable form.

The first shell and second shell portion are applied onto the tri-layer core according to the following process: A laboratory scale thermal cycle molding module as described in U.S. Patent Application 2003-0124183 A1, published July 3, 2003, the disclosure of which is incorporated herein by reference, having a diameter of 0.4219", is used to apply the first and second shell portions to the tri-layer core, and comprised a single mold assembly made from an upper mold assembly portion comprising an upper mold cavity, and a lower mold assembly portion comprising a lower mold cavity.

The lower mold assembly portion is first cycled to a hot stage at 85°C for 30 seconds. The first shell portion material (gastric fluid-resistant shell layer) is introduced into the lower mold cavity. A tri-layer core, prepared as described above, is then inserted into the lower mold cavity such that the first core portion, containing omeprazole, is inserted into the lower mold cavity. A blank upper mold assembly portion is mated with the lower mold assembly portion. The lower mold assembly is then cycled to a cold stage at 5°C for 60 seconds to harden the first shell portion. The blank mold assembly portion is removed from the lower mold assembly portion. The half-coated tri-layer core is ejected from the lower mold cavity. The upper mold assembly portion is cycled to a hot stage at 85°C for 30 seconds.

The second shell portion material (immediate release layer) is added to the upper mold cavity. The half-coated tri-layer core, coated with the first shell portion, is inserted into the upper mold cavity such that the uncoated tri-layer core containing antacid rested within the upper mold cavity. The lower mold assembly portion, which has been maintained at 5°C, is then mated with the upper mold assembly portion. The upper mold assembly portion is then cycled to a cold stage at 5°C for 120 seconds to harden the second shell portion. The lower mold assembly portion is then removed and the finished dosage form, a tri-layer core coated with two different shell portions, is ejected from the upper mold cavity. The weight gain due to the shell material (i.e. the difference in weight between the finished dosage form, and the tri-layer core) is recorded.

## Claims

1. A dosage form for oral administration comprising:
(a) at least one solid insert comprising a matrix having an acid-labile pharmaceutical active ingredient distributed therein;
(b) a barrier layer that is provided over the insert that is substantially free of acid-labile pharmaceutical active ingredient; and
(c) a gastric fluid-resistant layer that is provided over substantially the entire surface of the barrier layer;
wherein the barrier layer has a thickness of at least 200 microns at any point as measured from the outer surface of the at least one solid insert to the inner surface of the gastric fluid-resistant layer.

2. The dosage form according to claim 1, wherein the barrier layer is a compressed pharmaceutically acceptable powder material.

3. The dosage form according to claim 2, wherein the compressed barrier layer is substantially free of alkaline reacting compounds.

4. The dosage form according to claim 1, wherein at least one insert comprises an acid-labile proton pump inhibitor.

5. The dosage form according to claim 4, wherein at least one insert is substantially free of alkaline reacting compounds.

6. The dosage form according to claim 1, wherein the at least one solid insert comprises a matrix having at least one acid-labile pharmaceutical active ingredient and a dissolution enhancing vehicle.

7. The dosage form according to claim 1, wherein at least one insert consists essentially of a solid dispersion containing an acid-labile pharmaceutical active ingredient.

8. A dosage form for oral administration comprising:
(a) at least one solid insert comprising a matrix having an acid-labile pharmaceutical active ingredient distributed therein;
(b) a barrier layer that is provided over the insert that is substantially free of acid-labile pharmaceutical active ingredient; and
(c) a gastric fluid-resistant layer that is provided over substantially the entire surface of the barrier layer;
wherein the barrier layer has a minimum thickness that is greater than or equal to the smallest dimension of the at least one solid insert.

9. The dosage form according to claim 8, wherein the at least one solid insert comprises a matrix having at least one acid-labile pharmaceutical active ingredient and a dissolution enhancing vehicle.

10. A dosage form for oral administration comprising:
(a) a multi-compartment tablet comprising
(i) at least one solid insert comprising a matrix having an acid-labile pharmaceutical active ingredient;
(ii) a barrier layer that surrounds the insert and is substantially free of acid-labile pharmaceutical active ingredient;
(iii) an insulation layer that is provided over at least one surface of the barrier layer; and
(iv) a second active layer that is provided over the insulation layer;
wherein a coating is provided over said multi-compartment tablet and the coating has at least one gastric fluid-resistant section and at least one non-gastric juice fluid-resistant section.

11. A dosage form according to claim 10, wherein the barrier layer has a thickness of at least 200 microns at any point as measured from the outer surface of the at least one solid insert to the inner surface of the gastric fluid-resistant layer.

12. The dosage form according to claim 10, wherein the barrier layer is a compressed powder around the insert.

13. The dosage form according to claim 12, wherein the compressed barrier layer is substantially free of alkaline reacting compounds .

14. The dosage form according to claim 10, wherein at least one insert comprises an acid-labile proton pump inhibitor.

15. The dosage form according to claim 14, wherein at least one insert consists essentially of a solid dispersion containing an acid labile active ingredient.

16. The dosage form according to claim 10, wherein the at least one solid insert comprises a matrix having at least one acid-labile pharmaceutical active ingredient and a dissolution enhancing vehicle.

17. The dosage form according to claim 10, wherein the barrier layer consists essentially of a compressed powder pharmaceutically acceptable carbohydrate that does not react with an acid-labile active ingredient.

18. The dosage form according to claim 10, wherein the barrier layer has a minimum thickness that is greater than or equal to the smallest dimension of the at least one solid insert.

19. A method for production of a dosage form for oral administration comprising:
(a) forming a molded article as a insert that contains an acid-labile active ingredient;
(b) compressing a barrier layer around at least one insert containing the acid-labile active ingredient; and
(c) applying a gastric fluid-resistant layer over the barrier layer,
wherein the barrier layer has a thickness of at least 200 microns at any point as measured from the outer surface of the at least one solid insert to the inner surface of the gastric fluid-resistant layer.

20. A method according to claim 19 wherein the acid-labile active ingredient is a proton pump inhibitor.

21. A method for production of a dosage form for oral administration comprising:
(a) forming a multi-compartment core by
(i) providing at least one molded article as a insert that contains an acid-labile active ingredient
(ii) compressing a barrier layer around at least one insert;
(iii) applying an insulation layer over at least one surface of the barrier layer;
(iv) providing a second active layer over the insulation layer; and
(b) applying a coating over the multi-compartment core;
wherein a coating is provided over said multi-compartment tablet having at least one gastric fluid-resistant section and at least one non-gastric juice fluid-resistant section.

22. A method according to claim 21 wherein the acid-labile active ingredient is a proton pump inhibitor.

23. A method according to claim 21, wherein the barrier layer has a minimum thickness that is greater than or equal to the smallest dimension of the at least one solid insert.
